Europäisches Patentamt

(19) European Patent Office

Office européen des brevets

(11) Numéro de publication : **0 314 580 B1**

## (12) FASCICULE DE BREVET EUROPEEN

(45) Date de publication du fascicule du brevet :
**28.08.91 Bulletin 91/35**

(51) Int. Cl.⁵ : **A61K 31/00, A61K 31/06, // A61K37/02**

(21) Numéro de dépôt : **88402734.3**

(22) Date de dépôt : **28.10.88**

(54) **Application de groupes nitrophényles à la stimulation des capacités d'incorporation d'un médicament dans les cellules sensibles de l'hôte.**

(30) Priorité : **30.10.87 FR 8715127**

(43) Date de publication de la demande :
**03.05.89 Bulletin 89/18**

(45) Mention de la délivrance du brevet :
**28.08.91 Bulletin 91/35**

(84) Etats contractants désignés :
**AT BE CH DE ES FR GB GR IT LI LU NL SE**

(56) Documents cités :
**CHEMICAL ABSTRACTS, vol. 90, no. 7, 12 février 1979, page 54, résumé no. 48526m, Columbus, Ohio, US; K. MALBERG et al.: "6-(2,4-Dinitrophenyl)mercaptopurine, a stronger immunosuppressive drug than 6-mercaptopurine to primary humoral T cell dependent immuno response of mice", & ACTA BIOI. MED. GER. 1978, 37(4), 645-54**

(56) Documents cités :
**CHEMICAL ABSTRACTS, vol. 90, no. 23, 4 juin 1979, page 482, résumé no. 184730a, Columbus, Ohio, US; D. INBAR et al.: "Soluble trinitrophenylated proteins and trinitrophenylated cells as specific inhibitors of target cell lysis by cytotoxic T lymphocytes", & CELL. IMMUNOL. 1979, 42(2), 298-307**

(73) Titulaire : **INSTITUT PASTEUR**
**28, rue du Docteur Roux**
**F-75724 Paris Cédex 15 (FR)**

(72) Inventeur : **Avrameas, Eustrate-Stratis**
**1, rue Sarasate**
**F-75015 Paris (FR)**
Inventeur : **Matsiota-Bernard, Panayota**
**12 Square Beethoven**
**F-78330 Fontenay Le Fleury (FR)**
Inventeur : **Ternynck, Thérèse**
**39 bld Garibaldi**
**F-75015 Paris (FR)**

(74) Mandataire : **Gutmann, Ernest et al**
**S.C. Ernest Gutmann - Yves Plasseraud 67, boulevard Haussmann**
**F-75008 Paris (FR)**

EP 0 314 580 B1

## Description

La présente invention a pour objet l'application des groupes nitrophényles à la modification de principes actifs de médicaments afin de favoriser leur incorporation aux cellules sensibles de l'hôte auquel le médicament est destiné.

Par groupes nitrophényles on entend les molécules possédant des groupes dinitro ou trinitro-phényles et leurs dérivés.

Par cellule sensible on entend toute cellule de l'hôte constituant une cible reconnaissant des groupes nitrophényles. De telles cellules cibles sont notamment les lymphocytes T ou B, ou les thymocytes les fibroblastes, les cellules rénales... Ces cellules sont aussi sensibles pour certains agents pathogènes.

Il a déjà été observé que l'interaction in vivo entre un agent pathogène, par exemple un virus infectieux et les cellules sensibles correspondantes, se manifeste par une modification du taux d'anticorps naturels (AcN) chez l'hôte atteint de la maladie infectieuse ou autoimmune correspondante. Ces AcN sont des anticorps contenus dans les sérums et les liquides biologiques des humains et des animaux, capables de reconnaître des antigènes (Ag) du "soi" et du "non-soi". Ces AcN existent dans les liquides biologiques alors même que les hommes et les animaux n'ont pas été préalablement intentionnellement immunisés contre un agent pathogène déterminé.

La polyspécificité des AcN et le fait que souvent ils possèdent une haute affinité pour un antigène donné (T. Ternynck, S.Avrameas, Immunol. Rev. 1986) leur permet de reconnaître plusieurs antigènes présents chez des agents pathogènes. Il est à noter par ailleurs que parmi ces AcN, certains possèdent une activité du type anti-dinitrophényle (DNP) ou anti-trinitrophényle (TNP) et que dans plusieurs situations pathologiques (maladie autoimmunes et infections virales) le titre de ces anticorps (anti-NP) sériques est significativement modifié (P. Matsiota et al. Clin. exp. Immunol., 1987, Ann. Inst. Pasteur 1987).

Pour des commodités de langage, on regroupera sous le terme NP, les molécules contenant des groupes nitrophényles possédant 1, de préférence 2 ou 3 groupes nitro. De façon préférée, les molécules NP sont des nitrophénols. D'autres molécules intéressantes sont plus particulièrement le 2,4-dinitrophényle (DNP), ou le 2,4,6-trinitrophényle (TNP).

On a plus particulièrement remarqué que le taux d'anticorps ayant une activité anti-NP (Ac anti-NP) est significativement plus élevé que le taux des autres anticorps naturels lors de ces mêmes maladies auto-immunes ou de ces infections virales. Ces résultats ont notamment fait l'objet de publications à la suite d'études faites dans le cadre d'infections causées par le virus du SIDA (HIV) ("Détection of natural auto-anti-bodies in the serum of anti-HIV-positive individuals.", autrement dit "Détection d'auto-anticorps naturel dans le sérum d'individus ayant une réaction positive anti-HIV". P. Matsiota et al. Ann. Inst. Pasteur/Immunol 1987, 138, 223-233.) et dans le cadre d'une étude concernant le lupus érythémateux ("Natural antibodies in systemic lupus erythematosus." autrement dit "Anticorps naturels du lupus érythémateux de l'organisme".P. Matsiota et al. Clin. exp. Immunol (1987) 69, 79-88).

Il avait déjà par ailleurs été observé par certains auteurs une augmentation de la sensibilité des cellules à certains agents (6-mercaptopurine, Chemical Abstracts 90 : 48526 m, (1979), BSA, ibidem 90 : 184730 a, (1979)) lorsqu'ils étaient modifiés par un groupe nitrophényl. Toutefois, ce groupe était chaque fois lié directement à l'agent par une simple liaison covalente.

L'invention découle de la découverte faite par les inventeurs que la réalisation d'une mise en contact -in vitro ou in vivo- des cellules sensibles à un agent pathogène, avec des dérivés de NP reconnus par des anticorps anti-NP s'accompagnait dans la plupart des cas de l'internalisation d'une partie au moins de ces NP, dans les cellules sensibles correspondantes.

En d'autres termes, l'invention met à profit la capacité des NP mise en évidence par les inventeurs, à pénétrer dans des cellules sensibles, tout en étant susceptibles d'y entraîner le principe actif d'un médicament, dans la mesure où celui-ci avait auparavant été couplé chimiquement à un NP, notamment au niveau de la position C1 du cycle phényle ou au niveau de la position phénol du NP. En d'autres termes, le NP peut jouer le rôle d'un vecteur — ou d'une "locomotive" — capable d'induire ou de favoriser l'introduction de ce principe actif de médicament dans les cellules sensibles dans lesquelles il peut exercer les actions biologiques qui sous-tendent les effets thérapeutiques de ces principes actifs.

L'invention concerne par conséquent des conjugués réalisés par couplage chimique entre un NP, d'une part, et le principe actif de médicament d'autre part, dont la reconnaissance ou l'internalisation dans des cellules sensibles est recherchée.

L'invention vise plus particulièrement des composés tels que décrits ci-dessus, dans lesquels le principe actif de médicament est constitué par tout composé ayant une activité thérapeutique par exemple un agent antiviral, un agent antibactérien ou un agent cytostatique capables d'exercer — in vivo — leurs activités thérapeutiques respectives, notamment au niveau des cellules sensibles affectées.

Dans un mode de réalisation préféré des conjugués selon l'invention, le principe actif de médicament est couplé par une liaison covalente au vecteur constitué par un NP, au niveau de la position C1 du cycle phényle ou au niveau de la position phénol de celui-ci.

Il doit être remarqué que le couplage d'un NP, plus particulièrement au niveau de la position C1 du cycle phényle ou au niveau de sa position phénol, avec un principe actif de médicament, assure en même temps la neutralisation d'éventuelles propriétés toxiques in vivo de la molécule NP non modifiée, sans que soit affectée sa capacité à être reconnue par les anticorps anti-NP.

D'une façon générale, la conjugaison entre une molécule NP et le principe actif de médicament choisi peut être réalisée par toute réaction chimique appropriée mettant en oeuvre la position C1 du cycle phényle du NP ou sa position phénol d'une part, et une fonction réactive portée par le principe actif de médicament d'autre part. Par exemple, le principe actif de médicament porte une fonction amine, cas dans lequel on peut avoir recours à toute technique appropriée pour coupler entre elles cette fonction amine avec la fonction hydroxyle du NP. Lorsque la molécule de modification porte une fonction carboxyle on peut procéder à une réaction d'estérification.

Par exemple, les agents de condensation peuvent être constitués par des carbodiimides lorsque les fonctions complémentaires sont respectivement constituées par des groupes carboxyle et amine, ou vice-versa, par exemple le p-toluène-sulfonate de N-cyclohexyl-N-β-(N-méthyl-morpholino-éthyl)-carbodiimide et le chlorhydrate de (3-méthyl)-aminopropyl-carbodiimide ou le dicyclohexyl-carbodiimide. La réaction ester alors conduite dans des conditions usuelles dans le chimie des protéines.

Les agents de condensation peuvent encore être constitués par un 6-maléimido-caproïque-acyl-N-hydroxy-succinimide-ester ou le N-succinimidyl-3-(2-pyridyl-dithio)-propionate (SPDP), lorsque les fonctions complémentaires sont respectivement, au départ, un groupe sulfhydryle et un groupe amine ou vice-versa.

Des conditions de réaction appropriées sont encore celles décrites dans les articles suivants : J. CARLSSON et coll. Biochem. J. (1978) 173, 727-737 ou P.E. THORPE et coll. dans Euro. J. Biochem. (1981), 116, 447-454.

La compatibilité du principe actif de médicament avec la capacité de la molécule NP conjuguée à ce principe, peut être vérifiée par un test in vitro comprenant la mise en contact de ce conjugué avec des anticorps anti-NP préalablement immobilisés sur un support solide insoluble dans des conditions permettant la formation éventuelle d'un complexe immunologique entre des anticorps anti-NP et le conjugué et la détection de ce complexe immunologique.

De même, la compatibilité de la conjugaison avec la conservation de l'activité thérapeutique du principe actif de médicament peut être vérifiée, plus particulièrement lorsque ce principe exerce cet effet sur les mêmes cellules sensibles, par l'observation de l'effet pharmacologique de conjugué vis-à-vis de ces cellules sensibles par comparaison avec celles du principe actif de médicament non couplé.

On peut aussi mesurer le taux de principe actif internalisé dans les cellules, en particulier chaque fois que le principe actif se manifeste par une activité susceptible d'être dosée et qui n'est pas affectée par la conjugaison. Un test comprend alors, après la mise en contact in vitro du conjugué formé avec des cellules sensibles, la séparation des cellules et leur récupération hors du milieu dans lequel la susdite mise en contact avait été réalisée et le dosage (le cas échéant après rupture des parois cellulaires desdites cellules) de l'activité dosable retenue par ces cellules.

La conservation de la capacité des NP à réagir avec des cellules sensibles, après leur couplage avec le susdit principe actif de médicament peut alors être vérifiée par exemple par la mise en oeuvre d'un test comprenant la mise en contact, in vitro, de ce conjugué avec des cellules sensibles et la détection du taux d'incorporation dudit conjugué à ces cellules sensibles et, le cas échéant, la comparaison du taux d'incorporation observé avec le taux d'incorporation dans les mêmes cellules sensibles, dans les mêmes conditions selon le cas, du même NP, cependant non couplé, au principe actif de médicament.

L'invention concerne aussi des compositions pharmaceutiques associant le conjugué selon l'invention. Pour ce qui est de l'utilisation de ces compositions pharmaceutiques, il a lieu de noter que la détermination de doses appropriées sera effectuée dans chaque cas, selon la nature du principe actif de médicament entrant dans le conjugué selon l'invention et la nature de la pathologie à combattre. Les doses appropriées devront naturellement aussi être évaluées par le clinicien, au vu des sensibilités différentes des patients à la thérapeutique choisie.

D'autres caractéristiques techniques de l'invention apparaîtront dans l'exemple illustratif qui suit. Cet exemple a essentiellement pour but d'illustrer les capacités que possèdent les NP modifiés à s'intégrer à des cellules sensibles, notamment lorsque celles-ci sont sollicitées par un agent agresseur ou pathogène, d'où également le bien fondé de l'idée sur laquelle l'invention repose, à savoir la capacité des NP eux-mêmes à servir de vecteur permettant l'apport sélectif de principes actifs de médicaments aux cellules cibles qui doivent être atteintes.

## EXEMPLE I

Effet des dérivés NP sur la prolifération des lymphocytes de souris

### a/ Préparation des dérivés NP

Le TNP a été couplé à la sérumalbumine bovine (BSA) par la méthode de Little & Eisen : 1 ml de BSA à 20 mg/ml dans le tampon carbonate-bicarbonate, 0,1 M, pH 9,5, est ajouté à 1 ml d'une solution d'acide trinitrobenzène sulfonique à 2 mg/ml dans le même tampon. La réaction est arrêtée après 30 à 120 minutes selon la substitution voulue par élimination de l'excès de réactif sur échangeur d'ions (DOWEX 1 × 4) puis dialyse. Des préparations de BSA substituée avec soit 14 ou 30 molécules de TNP par molécule de BSA ($TNP_{14}$-BSA ou $TNP_{30}$-BSA) ont été utilisées.

Le DNP-Glycine (ENP-Gly) et le DNP-Lysine (DNP-Lys) proviennent des Laboratoires Sigma et sont solubilisés directement dans le milieu de culture.

### b/ Préparation des populations cellulaires

Les cellules proviennent de rate ou de thymus de souris. Les souris sont tuées par dislocation cervicale et leur rate ou leur thymus prélevé stérilement sont déposés dans du milieu RPMI. Les cellules sont dissociées, lavées une fois avec du milieu puis les globules rouges sont lysés au chlorure d'ammonium (0,9%) pendant 1 minute à froid puis les cellules sont relavées dans le milieu complet.

Les sous-populations de cellules B ou T sont ensuite séparées comme suit :

— Sous-population T : les cellules T sont obtenues à partir de la population de cellules de la rate par élimination des cellules portant des Ig à leur surface (sous-population B) sur des anticorps anti-Ig de souris immobilisés soit sur des billes magnétiques (Magnogel) soit sur des boites de Pétri.

— Sous-population B : les cellules B sont obtenues après détachement mécanique des supports sur lesquels elles étaient fixées par l'intermédiaire des anticorps anti-Ig (après l'isolement des cellules T), ou après élimination de la sous-population des cellules T de la population totale des cellules de rate par un traitement avec un anticorps anti-cellules T suivi de complément de cobaye.

— Thymocytes : les thymocytes sont dissociés dans le RPMI et les cellules isolées sont lavées dans le milieu complet avant d'être mises en culture.

### c/ Culture des cellules

Le milieu de culture utilisé est du RPMI 1640 contenant du sérum de veau foetal (5%), du pyruvate de sodium (1 mM), de la L-glutamine (1 mM), du 2-mercaptoethanol (0,05 mM), de la pénicilline (100 μl/ml) et de la streptomycine (50 μg/ml).

$2 \times 10^5$ cellules de chaque population cellulaire ou $4 \times 10^5$ de cellules de rate non fractionnées sont distribuées sous un volume final de 200 μl dans des boîtes de culture de 96 puits à fond plat en présence ou non des différents stimulateurs (ou mitogènes) (40 μg/ml de lipopolysaccharide W.S.Typhosal 2 μg/ml de concanavaline A, ou des surnageants de culture contenant les anticorps monoclonaux à différentes dilutions ou une solution de TNP-BSA à des concentrations finales allant de 200 ng à 200 μg/ml). Les cultures sont effectuées pendant 48 à 96 heures à 37°C dans une atmosphère de 5% de $CO_2$.

### d/ Mesure de la prolifération des cellules

Six à huit heures avant la fin de la culture, 100 μl de surnageant sont prélevés de chaque puits de culture afin d'être testés quant à leur contenu en anticorps. 10μl d'une solution de ³H-thymidine (activité spécifique = 5 μCi = 185 GBq/mM) à 50 μCi/ml sont ajoutés à la culture qui est ensuite poursuivie. Les cellules de chaque puits sont collectées sur du papier filtre à l'aide d'un collecteur automatique et, après séchage, les filtres sont comptés dans un liquide scintillant.

Les résultats obtenus sont rapportés dans le tableau I pour ce qui concerne l'action des dérivés NP sur des cellules données.

TABLEAU I

Effets des dérivés NP sur la prolifération des lymphocytes de la souris.

Le pourcentage d'augmentation (+) ou d'inhibition (–) est calculé par rapport aux chiffres obtenus avec le milieu seul (M) ou additionné de lipopolysaccharide (LPS) ou de concanavaline (con A).

\*Après traitement des cellules avec les dérivés NP pendant 48 heures, les mitogènes concanavaline A (con A) et lipopolysaccharide (LPS) sont ajoutés aux cellules et la culture poursuivie pendant 24 à 48 heures.

| Dérivés NP | TNP-BSA | | | DNP-Glycine | | | TNP-Lysine | | |
|---|---|---|---|---|---|---|---|---|---|
| | M | conA* | LPS* | M | conA* | LPS* | M | conA* | LPS* |
| **Cellules de rate** | | | | | | | | | |
| 200µg/ml | +50 | +46 | +30 | -64 | -50 | -60 | 0 | -6 | -3 |
| 50 | +30 | +30 | +20 | -30 | 0 | -20 | 0 | 0 | 0 |
| 12 | +10 | +28 | 0 | -10 | -30 | 0 | 0 | 0 | 0 |
| **Cellules B** | | | | | | | | | |
| 200µg/ml | +100 | nt | +18 | -90 | nt | -80 | -50 | nt | nt |
| 50 | +50 | nt | 0 | -60 | nt | -64 | -30 | nt | nt |
| 12 | 0 | nt | 0 | -50 | nt | -40 | -50 | nt | nt |
| 3 | 0 | nt | 0 | -25 | nt | -7 | -40 | nt | |
| **Cellules T** | | | | | | | | | |
| 200µg/ml | +30 | -50 | nt | +4 | -45 | nt | +32 | -50 | nt |
| 50 | +18 | -25 | nt | +30 | -15 | nt | +20 | -20 | nt |
| 12 | +20 | -20 | nt | 0 | -4 | nt | 0 | -20 | nt |
| 3 | 0 | -40 | nt | 0 | -5 | nt | 0 | -10 | nt |
| 0,7 | 0 | -50 | nt | +50 | 0 | nt | 0 | 0 | nt |

CONCLUSION

Ces résultats démontrent l'existence d'une activité des dérivés NP sur la prolifération des cellules lymphocytaires. Les cellules lymphocytaires sont donc parmi les cellules sensibles qui peuvent être visées par les procédés selon l'invention.

EXEMPLE II

Etude de l'effet des dérivés NP et des anticorps anti-TNP sur les lymphocytes de souris et leur interaction dans les réactions immunes.

La réaction immune (réaction lymphocytaire mixte ou MLR), qui intervient dans les expériences ci-dessous mentionnées, est basée sur la possibilité qu'ont les lymphocytes d'être stimulés par les antigènes d'histocompatibilité de cellules histo-incompatibles. Les lymphocytes, incubés avec des cellules (lymphocytes irradiés ou cellules dendritiques) portant des antigènes d'histocompatibilité différents se transforment et prolifèrent. La prolifération est mesurée par l'incorporation de thymidine tritiée. La présence de TNP/BSA dans les cultures (pendant les 5 jours de culture ou 48 heures avant la fin) modifie la prolifération par une augmentation ou non du nombre de cpm mesurés.

a/ Le TNP/BSA augmente les réactions lymphocytaires mixtes : cette stimulation est dépendante de la dose. La dose optimale varie selon les individus (entre 2,5 et 25 µg/ml). L'amplitude de la réaction varie selon les lignées et selon les individus.

```
     Augmentation des cpm par l'addition de TNP/BSA
              Quantité de TNP/BSA (µg/ml)
```

| | 250 | 25 | 2,5 | 0,25 | 0,025 | 0,003 |
|---|---|---|---|---|---|---|
| **H2-k/H2-d** | | | | | | |
| CBA n°1/DBA2 | 57000 | 68000 | 69000 | 36000 | 14000 | 12000 |
| CBA n°2/DBA2 | 44000 | 54000 | 55000 | 22000 | 0 | 0 |
| CBA/BALB | 29000 | 72000 | 75000 | 44000 | 40000 | 25000 |
| | | | | | | |
| **H2-k/H2-b** | | | | | | |
| CBA n°1/B6 | 62000 | 95000 | 100000 | 86000 | 103000 | 68000 |
| CBA n°2/B6 | 11000 | 21000 | 27000 | 12000 | 29000 | 0 |
| | | | | | | |
| **h2-d/H2-b** | | | | | | |
| DBA2/B6 | 23000 | 16000 | 11000 | 85000 | 71000 | 60000 |
| BALB/B6 | 2000 | 40000 | 12000 | 4000 | 3000 | 0 |
| DBA n°1/B6 | 21000 | 4000 | 1000 | 1000 | 1000 | 2000 |
| n°2/B6 | 24000 | 7000 | 4000 | 300 | 3000 | 5000 |
| n°3/B6 | 3000 | 8000 | 1000 | 0 | 0 | 0 |
| n°4/B6 | 9000 | 4000 | 0 | 0 | 0 | 0 |
| | | | | | | |
| **H2-b/H2-d** | | | | | | |
| B6 n°1/DBA2 | 20000 | 14000 | 82000 | 42000 | 62000 | 0 |
| B6 n°1/DBA2 | 34000 | 18000 | 8000 | 4000 | 6000 | 7000 |
| B6/BALB | 55000 | 10000 | 3000 | 7000 | 6000 | 0 |

b/ Le TNP/BSA stimule les réactions lymphocytaires syngéniques. La dose optimale et l'amplitude de la réaction varient selon les individus.

```
              Quantité de TNP/BSA (µg/ml)
```

| | 250 | 25 | 2,5 | 0,25 | 0,03 | 0,003 |
|---|---|---|---|---|---|---|
| **H2-d/H2-d** | | | | | | |
| BALB/C/DBA2 | 35000 | 0 | 2000 | 8000 | 15000 | 6000 |

```
2/ Les anticorps anti-TNP  :a/ ont une action sur la
réaction lymphocytaire mixte
H2-b/H2-d
C57B6
+anti-TNP        7000    3000    2000    2000    0
/DBA2 +
anti-TNP        33000   18000   13000   11000   3000
b/ n'ont pas d'effet sur la réaction lymphocytaire
syngénéique BA1B/C/DBA2 (mais les anticorps proviennent
de la souris BALB/C)
BALB/C n° 1
+anti-TNP        5000    9000    1000    0
/DBA2 +
anti-TNP        0       3000    0       0
BALB/C n° 2
+anti-TNP        133000  6000    2000    2000
/DBA2 +
anti-TNP        -1000   0       -2000   0
```

CONCLUSIONS : Les dérivés NP modifient les réactions de reconnaissance des lymphocytes en augmentant la stimulation entre lymphocytes histo-incompatibles. Les doses optimales et l'amplitude de la réaction varient selon les lignées et selon les individus.

Les anticorps anti-TNP ont un effet d'amplification significatif sur ces réactions sauf sur les souris BALB dont proviennent les anticorps.

## Revendications

1. Conjugué résultant d'un couplage entre un principe actif de médicament et un nitrophényle, capable d'induire ou de favoriser l'introduction de ce principe actif de médicament dans les cellules sensibles à un agent pathogène dans lesquelles il peut exercer les actions biologiques qui sous-tendent les effets thérapeutiques de ces principes actifs à l'égard de cet agent pathogène.

2. Conjugué selon la revendication 1, caractérisé en ce que le conjugué fait intervenir un nitrophényle et que la liaison covalente intervient en C1 du cycle phényle ou au niveau de sa position phénol.

3. Conjugué selon la revendication 2, caractérisé en ce que le nitrophényle est un 2,4-dinitrophényle.

4. Conjugué selon la revendication 2, caractérisé en ce que le nitrophényle est un 2,4,6-trinitrophényle.

5. Composition pharmaceutique caractérisée en ce qu'elle contient un conjugué tel que défini dans l'une quelconque des revendications 1 à 3, en association avec un véhicule pharmaceutiquement acceptable.

6. Application d'un conjugué selon l'une quelconque des revendications 1 à 4, à la production d'un médicament pour le traitement d'une affection semblable à celle qui peut être traitée par le principe actif du médicament intervenant dans le conjugué, mais à l'état libre.

## Patentansprüche

1. Aus einer Kopplung zwischen einem aktiven Bestandteil eines Medikaments und einem Nitrophenyl entstandenes Konjugat, das in der Lage ist, diesen aktiven Bestandteil eines Medikaments in für ein pathogenes Agens empfindliche Zellen einzuführen oder seine Einführung zu begünstigen, in denen er die biologischen Wirkungen aus-üben kann, die den therapeutischen Wirkungen dieser aktiven Bestandteile im Hinblick auf dieses pathogene Agens zu Grunde liegen.

2. Konjugat nach Anspruch 1, dadurch gekennzeichnet, daß das Konjugat ein Nitrophenyl zum Einsatz bringt und daß die kovalente Bindung am $C_1$-Atom des Phenylrings oder an der Stelle seiner Phenol-Position vorliegt.

3. Zusammensetzung nach Anspruch 2, dadurch gekennzeichnet, daß das Nitrophenyl ein 2,4-Dinitrophenyl ist.

4. Zusammensetzung nach Anspruch 2, dadurch gekennzeichnet, daß das Nitrophenyl ein 2, 4, 6-Trinitrophenyl ist.

5. Arzneimittel, dadurch gekennzeichnet, daß es ein Konjugat wie in einen der Ansprüche 1 bis 3 definiert in Verbindung mit einem pharmazeutisch verträglichen Träger enthält.

6. Verwendung eines Konjugates nach einem der Ansprüche 1 bis 4 zur Herstellung eines Medikaments zur Behandlung einer Erkrankung ähnlich der, die mit dem in dem Konjugat zum Einsatz gelangenden aktiven Bestandteil des Medikaments, jedoch in freiem Zustand, behandelt werden kann.

## Claims

1. Conjugate resulting from a coupling between an active principal of drug and a nitrophenyl, capable of inducing or promoting the introduction of this active principle of drug in cells susceptible to a pathogenic agent, in which it can exert the biological actions which underlie the therapeutic effects of these active principal with respect to this pathogenic agent.

2. Conjugate according to claim 1, characterized in that the conjugate involves a nitrophenyl and the covalent linkage occurs at C1 of the phenyl ring or at the level of its phenol position.

3. Conjugate according to claim 2, characterized in that the nitrophenyl is a 2,4-dinitrophenyl.

4. Conjugate according to claim 2, characterized in that the nitrophenyl is a 2,4,6-trinitrophenyl.

5. Pharmaceutic composition characterized in that it contains a conjugate as defined in anyone of claims 1 to 3, in association with an acceptable pharmaceutical carrier.

6. Use of a conjugate according to anyone of claims 1 to 4, to the production of a drug for the treatment of a disease similar to that which may be treated by the active principle of the drug involved in the conjugate, but in a free state.